# EUROPÄISCHE PATENTSCHRIFT

(11) **EP 2 889 005 B1**
(45) Veröffentlichungstag und Bekanntmachung des Hinweises auf die Patenterteilung: **31.08.2016**
(21) Anmeldenummer: 13199690.2
(22) Anmeldetag: 27.12.2013
(51) Int. Cl.: A61B 17/00, A61B 18/14

(54) **Medizinisches Instrument mit verstellbarem Werkzeug**
Medical instrument with adjustable tool
Instrument médical avec outil réglable

(43) Veröffentlichungstag der Anmeldung: 01.07.2015
(73) Patentinhaber: ERBE Elektromedizin GmbH, 72072 Tübingen (DE)
(72) Erfinder: Mayer, Volker, 72072 Tübingen (DE); Voigtländer, Matthias, 72810 Gomaringen (DE)
(74) Vertreter: Rüger, Barthelt & Abel

(56) Entgegenhaltungen:
- EP-A1- 0 247 917
- EP-A2- 1 985 250
- EP-A2- 1 990 018
- WO-A1-95/04207
- JP-A- 2004 016 309
- JP-A- 2005 261 734
- JP-A- 2006 180 939

## Beschreibung

Die Erfindung betrifft ein medizinisches Instrument, insbesondere für ein Endoskop, wobei das Instrument einen verstellbar gelagerten Werkzeugteil aufweist.

Medizinische Instrumente, insbesondere für den endoskopischen Einsatz vorgesehene Instrumente mit verstellbaren Werkzeugteilen, sind bekannt, wie beispielsweise die EP 2 604 202 A1 zeigt. Das dort beschriebene endoskopische Instrument weist als verstellbar gehaltenen Werkzeugteil eine durch ein Röhrchen gebildete HF-Elektrode auf, die mit einem Kolben verbunden und hydraulisch zwischen zwei Endstellungen beweglich ist.

Die WO 2009/030324 A1 offenbart ein elektrochirurgisches Instrument für ein Endoskop mit einer Elektrode, die axial in distaler und proximaler Richtung beweglich ist. Um verschiedene Positionen festzulegen, ist eine Raste mit drei Rastpositionen vorgesehen.

Die US 8,048,073 B2 offenbart ein weiteres endoskopisches Instrument mit einer Elektrode, die als Werkzeug dient und mit einer Stellvorrichtung in mindestens zwei, in einer anderen Ausführungsform auch in drei verschiedene Axialpositionen überführt werden kann. Dazu ist die Elektrode mit einem Draht verbunden, der sowohl axiale Zug- und Schubbewegungen wie auch Drehbewegungen auf die Elektrode überträgt. Mit der Elektrode ist ein Stopper verbunden, der zwei sich in Radialrichtung erstreckende Nasen oder Flügel aufweist. Entsprechend sind an dem distalen Ende des Schlauchs Scheiben versehen, die einen der Stopperkontur angepassten Durchgang für den Stopper aufweisen. Die unrunden Durchgänge der Scheiben sind jeweils um eine Drehung gegeneinander versetzt, so dass der durch eine Scheibe axial hindurch geschobene Stopper an der nächsten Scheibe eine feste Anlage findet. Durch weiteres Drehen kann er wiederum bis zur nächsten Scheibe vorgeschoben werden. Zwischen den Scheiben hat der Stopper ein Axialspiel.

Die zweiteilige Form des Anspruchs 1 basiert auf US 8,048,073 B2. Der Schutzumfang der Erfindung wird durch den unabhängigen Anspruch 1 definiert. Die vorteilhaften Ausführungen sind durch die abhängigen Ansprüche gegeben.

Bei der Anwendung von elektrochirurgischen und anderen endoskopischen Instrumenten kommt es darauf an, das Werkzeug präzise zu positionieren. Differenzen zwischen der gewünschten Ausfahrweite einer Elektrode und der tatsächlichen Ausfahrweite können zu unterschiedlichen Anwendungsergebnissen führen. Auch wird häufig eine taktile Rückmeldung über die Position des Werkzeugs gewünscht.

Davon ausgehend ist es Aufgabe der Erfindung, ein medizinisches Instrument mit verbesserter Handhabbarkeit zu schaffen.

Diese Aufgabe wird mit dem Instrument nach Anspruch 1 gelöst.

Das erfindungsgemäße Instrument weist einen länglichen Schlauch bzw. ein biegsames Rohr auf, durch den bzw. das sich ein axial beweglicher und drehbeweglicher Draht erstreckt. Der Begriff Draht umfasst dabei alle länglichen Mittel, die Zugkräfte und wenigstens in beschränktem Maß auch Druckkräfte übertragen können sowie eine Drehsteifigkeit aufweisen, die ausreicht, um eine Drehung von dem proximalen Ende des Drahts auf sein distales Ende zu übertragen. An einem Ende dieses Drahts ist ein Werkzeugteil angebracht oder wird von dem Drahtende gebildet. Zur Festlegung der Axialposition dieses Werkzeugteils ist eine Axialpositioniereinrichtung vorgesehen, die in oder an dem distalen Ende des Schlauchs angeordnet und zwischen dem Draht oder dem Werkzeugteil sowie dem Schlauch wirksam ist. Die Axialpositioniereinrichtung ist von dem proximalen Ende des Instruments her steuerbar. Sie wird vorzugsweise durch eine kombinierte Dreh- und Schubbewegung bzw. Dreh- und Zugbewegung gesteuert. Dazu weist die Axialpositioniereinrichtung eine Kulissenführung mit einer Kulissennut auf. Diese gibt die Bewegung des Werkzeugteils vor und legt Axialpositionen desselben präzise fest. Außerdem gibt die Kulissenführung selbst bei relativ langen Instrumenten, d.h. mit langen Drähten, dem Bediener ein gutes taktiles Gefühl, d.h. eine taktile Rückmeldung für die erreichten Axialpositionen.

Der Draht, d.h. das Verschiebemittel kann als Hohldraht ausgebildet sein und somit zum Beispiel als Fluidleiter dienen. Beispielsweise kann der Werkzeugteil eine Fluidaustrittsöffnung aufweisen, die von dem Fluidleiter gespeist wird. Durch den Hohldraht können somit gewünschte Fluide in das Anwendungsgebiet eingeleitet werden, beispielsweise um fluidchirurgische Eingriffe durchzuführen.

Unabhängig davon kann der Draht aus Metall bestehen und als elektrischer Leiter dienen. Der Werkzeugteil kann somit als Elektrode dienen. Unabhängig davon kann er, wie oben erwähnt, als Kanüle ausgebildet sein.

Vorzugsweise hat der Werkzeugteil die Form eines länglichen Stifts, der koaxial zu dem distalen Ende des Schlauchs angeordnet sein kann. Bei einer bevorzugten Ausführungsform kann der Werkzeugteil in das Schlauchende eingezogen oder aus diesem heraus ragend positioniert sein. Insbesondere ist es möglich, dass die Axialpositioniereinrichtung auch Zwischenpositionen festlegt. Dem Anwender sind dadurch differenzierte Anwendungsmöglichkeiten gegeben.

Zu der Axialpositioniereinrichtung gehören vorzugsweise ein Kulissenträger, in dem die Kulissennut ausgebildet ist, sowie ein Kulissenstein, der in der Kulissennut beweglich angeordnet ist. Der Kulissenträger kann mit dem Draht verbunden und somit beweglich angeordnet sein. Der Kulissenstein ist in diesem Fall dem Schlauch zugeordnet. Die Anordnung kann jedoch auch umgekehrt getroffen sein. Es ist auch möglich, dass der Kulissenstein Teil des Werkzeugteils ist und mit diesem einstückig, nahtlos verbunden ist.

In einer weiteren Ausführungsform ist es möglich, dass die Kulissennut direkt in den Sondenschlauch integriert ist. Dadurch entfällt ein gesondertes Teil, das die Kulissennut enthält. Der Kulissenstein, der direkt am Werkzeug oder an einem separaten Teil ausgebildet ist, ist dann beweglich in der Kulissennut des Sondenschlauchs angeordnet.

Die Kulissennut kann verschiedene Formen aufweisen. Bei einer bevorzugen Ausführungsform ist sie hingegen als Mäandernut ausgebildet. Sie weist dazu in Axialrichtung verlaufende Abschnitte und dazwischen in Umfangsrichtung verlaufende Abschnitte auf. Der Kulissenstein kann in dieser Mäandernut bewegt werden, wobei der Bewegungsweg axiale und Umfangskomponenten hat. Kann er sich in Umfangsrichtung bewegen, ist seine Axialposition festgelegt. Kann er sich in Axialrichtung bewegen, ist seine Umfangsposition festgelegt. Der Bediener spürt eine deutliche Führung und hat ein klares Rastgefühl. Insbesondere kann er mit einer Drehstellbewegung die Axialposition des Werkzeugteils immer nur um eine Rastposition weiterschalten. Versehentliches Überspringen einer Rastposition ist bei der Mäandernut nicht möglich.

Weitere Details und Einzelheiten von Ausführungsformen der Erfindung sind Gegenstand der Zeichnung, der Beschreibung oder von Ansprüchen. Es zeigen:
Figur 1 ein endoskopisches Instrument, in ausschnittsweiser Ansicht.
Figur 2 das distale längs aufgeschnittene Ende des Instruments nach Figur 1, in schematisierter Ansicht,
Figur 3 einen Werkzeugteil sowie weitere Elemente des distalen Endes des Instruments nach Figur 1, in ausschnittsweiser Perspektivansicht,
Figur 4 die Abwicklung einer Kulissennut gemäß Figur 2, in schematisierter Darstellung,
Figur 5 und 6 eine abgewandelte Ausführungsform des erfindungsgemäßen Instruments mit abgewandelter Axialpositioniereinrichtung in längsgeschnittener bzw. perspektivischer Ansicht.

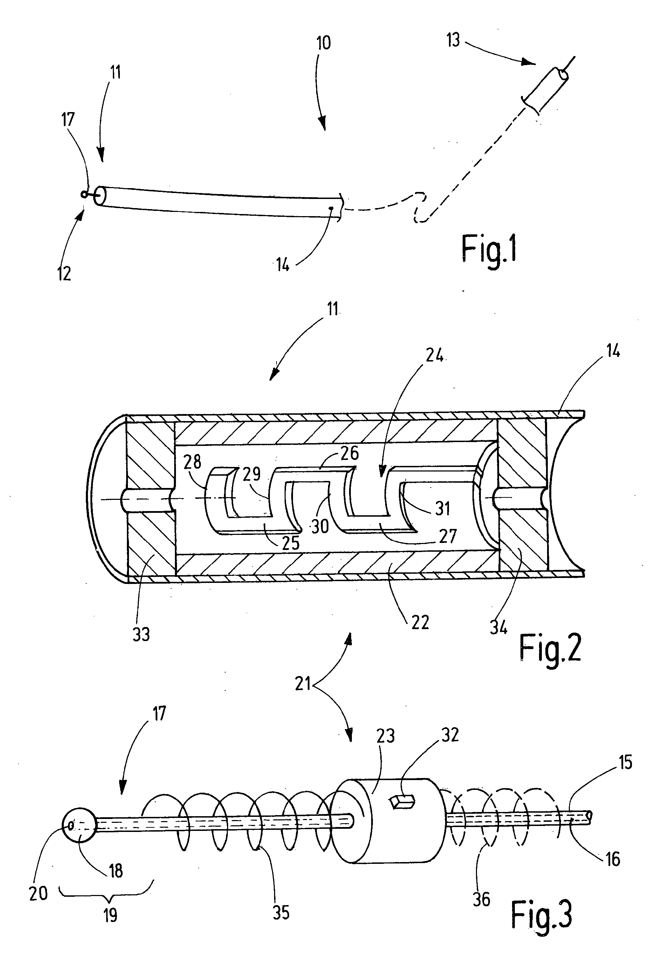

In Figur 1 ist ein medizinisches Instrument 10 veranschaulicht, das für den endoskopischen Einsatz vorgesehen ist. Dabei wurde die Darstellung auf die sich an das distale Ende 11 und das proximale Ende 13 anschließenden Abschnitte beschränkt. Als Beispiel für ein erfindungsgemäßes Instrument 10 dient hier ein Instrument, das zugleich elektrochirurgischen als auch fluidchirurgischen Einsatz gestattet. Dazu ist der aus dem distalen Ende 11 vorstehende Werkzeugteil 12 entsprechend bi- bzw. multifunktional ausgebildet. Es wird jedoch darauf hingewiesen, dass die Erfindung auch bei anderen Instrumenten anwendbar ist, die lediglich monofunktionale Werkzeugteile aufweisen.

Das Instrument 10 weist einen sich von dem proximalen Ende 13 zu dem distalen Ende 11 erstreckenden Schlauch 14 auf, der vorzugsweise einen geringen Durchmesser von beispielsweise lediglich wenigen Millimetern oder weniger aufweist.

Durch den Schlauch 14 erstreckt sich ein Draht 15, der im vorliegenden Ausführungsbeispiel als Hohldraht bzw. Kapillarrohr ausgebildet ist und einen inneren Fluidkanal 16 umschließt. Der Draht 15 kann auch ein Volldraht oder ein Litzendraht sein und unabhängig davon prinzipiell aus Metall, Kunststoff oder einem anderen geeigneten Material bestehen. Vorliegend besteht er aus Metall und bildet einen elektrischen Leiter, der mit einem HF-Generator verbindbar ist.

Der Draht 15 ist mit einem Werkzeugteil 17 verbunden, wie es beispielsweise aus Figur 1 und 3 hervorgeht. Der Werkzeugteil 17 kann durch das distale Ende des Drahts 15 oder durch ein gesondertes Element gebildet sein, das mit dem Draht 15 vorzugsweise koaxial fluchtend verbunden ist. Der Werkzeugteil 17 weist beispielsweise die Form eines Zylinderstifts auf, der an seinem freien Ende mit einem Isolator beispielsweise aus Keramik oder, wie dargestellt, einer Metallkugel 18 versehen sein kann. Der Werkzeugteil 17 und die Kugel 18 bilden zusammen eine Elektrode 19. An dem distalen Ende des Werkzeugteils 17, beispielsweise in der Metallkugel 18, kann eine Fluidaustrittsöffnung 20 angeordnet sein, an der der Fluidkanal 16 mündet. Der Werkzeugteil 17 kann damit, je nach Ausbildung und Fluiddruck, als Injektionsdüse, z.B. zur Gewebeunterspritzung, Parenchymauflösung oder dergleichen, oder als Kanüle dienen. Zugleich kann er, wenn er aus Metall ist, als Elektrode dienen, z.B. für HF-chirurgische Anwendungen. Die Elektrode 19 kann auch andere von einem Zylinderstift abweichende Formen aufweisen. So sind Hakenelektroden und auch andere an deren distalen Ende nicht symmetrisch geformte Elektroden möglich.

Zur Axialpositionierung des Werkzeugteils 17 dient eine Axialpositioniervorrichtung 21, zu der ein Kulissenträger 22 und ein Nutensteinträger 23 gehören. Bei der Ausführungsform der Axialpositioniervorrichtung 21 nach Figur 2 und 3 ist der Kulissenträger 22 beispielsweise eine Hülse bzw. ein Hohlzylinder, während der Nutensteinträger 23, beispielsweise als Zylinder oder als Scheibe oder anderweitig ausgebildet sein kann und jedenfalls in den Durchgang des Kulissenträgers 22 passt.

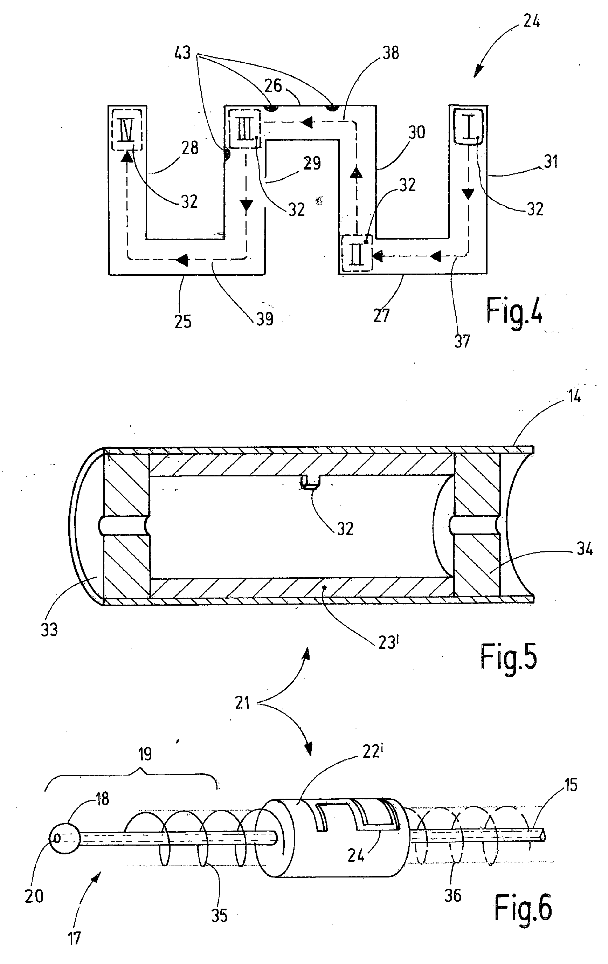

Der Kulissenträger 22 weist eine Kulissennut 24 auf, die vorzugsweise in Mäanderform angelegt ist, wie es aus Figur 2 und abstrahiert aus Figur 4a ersichtlich ist. Zu der Kulissennut 24 gehören mehrere Axialabschnitte 25, 26, 27, die wechselweise in Umfangsrichtung wie in Axialrichtung gegeneinander versetzt angeordnet sind und die sich in Axialrichtung erstrecken.

An jeden Axialabschnitt 25, 26, 27 schließen sich endseits jeweils Umfangsabschnitte 28, 29, 30, 31 an, die parallel zueinander orientiert sind und rechtwinklig zur Axialrichtung entlang des Umfangs verlaufen. Die Abstände zwischen den Umfangsabschnitten 28, 29, 30, 31 (und somit die Längen der Axialabschnitte 25, 26, 27) können einheitlich oder auch unterschiedlich festgelegt sein. Die Umfangsabschnitte 28 bis 31 legen bei der Axialpositioniereinrichtung 21 nach Figur 2 und 3 insgesamt vier verschiedene Axialpositionen fest. Außerdem begrenzen sie die Drehbewegung des Nutensteinträgers 23 in beiden Drehrichtungen. An einander entgegengesetzten Enden der Umfangsabschnitte 29, 30 schließen in unterschiedliche Axialrichtungen führende Axialabschnitte 25, 26 bzw. 26, 27 an.

Der Kulissennut 24 ist ein Kulissenstein 32 zugeordnet, der als radial von dem Nutensteinträger 23 weg ragender Fortsatz ausgebildet ist. Seine Größe ist vorzugsweise so bemessen, dass er mit geringem Spiel in die Kulissennut 24 passt.

Der Nutensteinträger 23 wie der Kulissenstein 32 kann einstückig aus jedem geeigneten Material, wie bspw. Kunststoff, Keramik, Metall oder dergleichen, ausgebildet sein. Gleiches gilt für den hülsenartigen Kulissenträger 22, wobei dieser auch zweistückig ausgeführt sein kann. Der Nutensteinträger 23 passt vorzugsweise mit geringem Radialspiel in die zylindrische Durchgangsöffnung des Kulissenträgers 22. Stirnseitig ist der Kulissenträger 22 jeweils von zwei Abschlussscheiben 33, 34 oder geeigneten anderen Abschlusselementen begrenzt, die jeweils einen Durchgang für den Draht 15 bzw. das Werkzeug 17 aufweisen. Es können bedarfsweise zwischen zumindest einer der Abschlussscheiben 33, 34 und dem Nutensteinträger 23 ein Federmittel, beispielsweise in Gestalt einer Druckfeder 35 oder 36 vorgesehen sein.

Bei einer zweistückigen Ausgestaltung des hülsenartigen Kulissenträgers 22 (nicht dargestellt) können die Abschlussscheiben 33, 34 Teil dieses sein. In diesem Falle kann der Kulissenträger 22 an seiner längsverlaufenden Symmetrieachse geteilt sein und an seinen Endbereichen jeweils zwei halbkreisförmige Endstücke aufweisen, die im montierten Zustand den Abschlussscheiben 33, 34 entsprechen.

Das insoweit beschriebene Instrument arbeitet wie folgt.

In Betrieb sitzt der Nutensteinträger 23 in dem Kulissenträger 22, wobei der Kulissenstein 32 an geeigneter Stelle innerhalb der Kulissennut 24 angeordnet ist. Die Druckfeder 35 spannt den Nutensteinträger 23 in einer Axialrichtung, hier in Einzugsrichtung vor. Die Verhältnisse sind schematisch in den Figuren 4a und 4b dargestellt. Der Kulissenstein 32 befindet sich in dem Umfangsabschnitt 31 bzw. dem quer zur Längsachse liegenden Abschnitt 42. Der Werkzeugteil 17 ist dadurch in einer vollständig zurückgezogenen Position arretiert.

Soll der Werkzeugteil 17 ein kleines Stück, beispielsweise einen Millimeter oder, je nach Abstand der Umfangsabschnitte 28 bis 31 voneinander etwas mehr ausgefahren werden, wird der Nutensteinträger 23 mittels des Drahts 15 gedreht und in distaler Richtung vorgeschoben, wie in Figur 4a, und 4b durch einen gestrichelten Pfeil 37 symbolisiert ist. Der Kulissenstein 32 kann dadurch in die gestrichelt gezeichnete Arretierungsposition II überführt werden. Ebenso kann er, wie ein weiterer Pfeil 38 bzw. 39 andeutet, wiederum durch eine kombinierte Dreh- und Schiebebewegung in die Arretierungsposition III bzw. IV überführt werden. Auf entgegen gesetztem Wege wird der Werkzeugteil 17 wieder eingezogen.

Eine sichere Arretierung des Kulissensteins 32 in der Arretierungsposition I, II, III, oder IV wird durch die in Figur 4a dargestellten Arretierungselemente 43 erreicht. Vorzugsweise sind diese Arretierungselemente 43 in den Bereichen angeordnet, in denen die Axialposition des Werkzeugteils 17 sichergestellt sein muss. Diese Arretierungselemente 43 können durch Vorsprünge oder Federelemente gebildet sein, welche den Querschnitt der Kulissennut 24 geringfügig reduzieren. Diese Vorsprünge können durch Prägeverfahren, spanabhebende Verfahren oder Spritzgussverfahren hergestellt werden.

Eine weitere Möglichkeit der sicheren Arretierung des Kulissensteins 32 in der Arretierungsposition I, II, III, oder IV kann durch die Einwirkung einer Torsionskraft erbracht werden. Diese Torsionskraft muss entgegen der Richtung aufgebracht werden, in der sich der an die Arretierungsposition I, II, III, oder IV folgende Abschnitt der Kulissennut 24 erstreckt.

Die Figuren 5 und 6 veranschaulichen eine abgewandelte Ausführungsform, die sich von der vorigen Ausführungsform durch die Ausbildung der Axialpositionierungsvorrichtung 21 unterscheidet. Ein den Kulissenträger 22 ersetzender Nutensteinträger 23' weist den nunmehr ortsfest angeordneten radial nach innen ragenden Kulissenstein 32 auf. Diesem ist die Kulissennut 24 zugeordnet, die in dem nunmehr mit dem Draht 15 verbundenen zylindrischen Kulissenträger 22' ausgebildet ist. Die Funktion stimmt jedoch mit der vorstehend beschriebenen Funktion des Instruments 10 nach den Figuren 2 und 3 überein, so dass auf die oben stehende Beschreibung entsprechend unter Zugrundelegung gleicher Bezugszeichen verwiesen wird.

Ein medizinisches, insbesondere für den endoskopischen Einsatz vorgesehenes Instrument 10 weist eine axial verstellbare Elektrode 19 oder einen sonstigen Werkzeugteil 17 auf, dem eine Axialpositioniervorrichtung 21 zugeordnet ist. Diese umfasst einen Kulissenträger 22 und einen Nutensteinträger 23, die an dem distalen Ende des Instruments 10 angeordnet sind. Die Axialpositioniervorrichtung 21 wird durch eine kombinierte Dreh- und Schiebe- bzw. Dreh- und Zugbewegung gesteuert und legt mehrere verschiedene Axialpositionen spielarm fest.

### Bezugszeichenliste:

- 10: Instrument
- 11: distales Ende
- 12: Werkzeugteil
- 13: proximales Ende
- 14: Schlauch
- 15: Draht
- 16: Fluidkanal
- 17: Werkzeugteil
- 18: Metallkugel
- 19: Elektrode
- 20: Fluidaustrittsöffnung
- 21: Axialpositioniervorrichtung
- 22, 22': Kulissenträger
- 23, 23': Nutensteinträger
- 24: Kulissennut
- 25 - 27: Axialabschnitte der Kulissennut 24
- 28 - 31: Umfangsabschnitte
- 32: Kulissenstein
- 33, 34: Abschlussscheiben
- 35, 36: Druckfeder
- I -IV: Arretierungspositionen
- 37 - 39: Pfeil

- 43: Arretierungselement

## Patentansprüche

1. Medizinisches endoskopisches Instrument (10),
mit einem länglichen Schlauch (14), durch den sich ein axialbeweglicher und drehbeweglicher Draht (15) erstreckt,
mit einem verstellbar gehaltenem Werkzeugteil (17) das mit dem distalen Ende des Drahts (15) verbunden oder von diesem gebildet ist,
mit einer Axialpositioniereinrichtung (21), die in oder an dem distalen Ende des Schlauchs (14) angeordnet und zwischen dem Draht (15) und dem Schlauch (14) wirksam ist,
wobei
die Axialpositioniereinrichtung (21) eine Kulissenführung mit einer Kulissennut (24) aufweist, die aus aneinander anschließenden geraden Abschnitten besteht,
**dadurch gekennzeichnet,**
**dass** die Kulissennut (24) parallel zu dem Draht (15) orientierte Axialabschnitte (25, 26, 27) und rechtwinklig zu diesen orientierte Umfangsabschnitte (28, 29, 30, 31) aufweist, die einander abwechselnd angeordnet sind und eine Mäandernut bilden.

2. Instrument nach Anspruch 1, **dadurch gekennzeichnet, dass** der Draht (15) ein Hohldraht ist.

3. Instrument nach einem der vorstehenden Ansprüche, **dadurch gekennzeichnet, dass** der Werkzeugteil (17) eine Elektrode (19) ist.

4. Instrument nach einem der vorstehenden Ansprüche, **dadurch gekennzeichnet, dass** zu der Axialpositioniereinrichtung (21) ein Kulissenträger (22), in dem die Kulissennut (24) ausgebildet ist, und ein Kulissenstein (32) gehören, der in der Kulissennut (24) längs- und drehbeweglich angeordnet ist.

5. Instrument nach Anspruch 4, **dadurch gekennzeichnet, dass** der Kulissenträger (22) mit dem Draht (15) verbunden und in oder an dem distalen Ende des Schlauchs (14) längs- und drehbeweglich angeordnet ist.

6. Instrument nach Anspruch 5, **dadurch gekennzeichnet, dass** der Kulissenstein (32) in oder an dem distalen Ende des Schlauchs (14) ortsfest angeordnet ist.

7. Instrument nach einem der vorstehenden Ansprüche, **dadurch gekennzeichnet, dass** der Kulissenträger (22) mit dem distalen Ende des Schlauchs (14) ortsfest verbunden ist.

8. Instrument nach Anspruch 1, **dadurch gekennzeichnet, dass** die Kulissennut (24) in dem Schlauch (14) ausgebildet ist.

9. Instrument nach Anspruch 4, **dadurch gekennzeichnet, dass** der Kulissenstein (32) mit dem Draht verbunden und in dem distalen Ende des Schlauch (14) längs- und drehbeweglich angeordnet ist.

10. Instrument nach Anspruch 1, **dadurch gekennzeichnet, dass** alle Axialabschnitte (25, 26, 27) zueinander fluchtend angeordnet sind.

11. Instrument nach Anspruch 1, **dadurch gekennzeichnet, dass** die Kulissennut (24) wenigstens ein Arretierungselement (43) aufweist.

12. Instrument nach Anspruch 1, **dadurch gekennzeichnet, dass** alle Umfangsabschnitte (28, 29, 30, 31) einheitlich lang ausgebildet sind.

## Claims

1. Medical endoscopic instrument (10),
having an elongated tube (14) through which an axially moveable and rotationally moveable wire (15) extends,
having a tool part (17) which is held adjustably and which is connected to the distal end of the wire (15) or is formed by this,
having an axial positioning device (21) which is arranged in or on the distal end of the tube (14) and is active between the wire (15) and the tube (14),
wherein
the axial positioning device (21) has a sliding block guide having a sliding block groove (24) which consists of straight sections which connect to one another,
**characterised in that**
the sliding block groove (24) has axial sections (25, 26, 27) which are orientated in parallel to the wire (15) and peripheral sections (28, 29, 30, 31) which are orientated at a right angle to these and which are arranged to be alternating and form a meandering groove.

2. Instrument according to claim 1, **characterised in that** the wire (15) is a hollow wire.

3. Instrument according to one of the preceding claims, **characterised in that** the tool part (17) is an electrode (19).

4. Instrument according to one of the preceding claims, **characterised in that** a sliding block support (22), in which the sliding block groove (24) is formed, and a sliding block (32), which is arranged to be longitudinally and rotationally moveable in the sliding block groove (24), are included in the axial positioning device (21).

5. Instrument according to claim 4, **characterised in that** the sliding block support (22) is connected to the wire (15) and is arranged in or on the distal end of the tube (14) to be longitudinally and rotationally moveable.

6. Instrument according to claim 5, **characterised in that** the sliding block (32) is arranged in or on the distal end of the tube (14) to be fixed in place.

7. Instrument according to one of the preceding claims, **characterised in that** the sliding block support (22) is connected to the distal end of the tube (14) to be fixed in place.

8. Instrument according to claim 1, **characterised in that** the sliding block groove (24) is formed in the tube (14).

9. Instrument according to claim 4, **characterised in that** the sliding block (32) is connected to the wire and is arranged in the distal end of the tube (14) to be longitudinally and rotationally moveable.

10. Instrument according to claim 1, **characterised in that** all axial sections (25, 26, 27) are arranged to align with one another.

11. Instrument according to claim 1, **characterised in that** the sliding block groove (24) has at least one locking element (43).

12. Instrument according to claim 1, **characterised in that** all peripheral sections (28, 29, 30, 31) are formed to be consistently long.

## Revendications

1. Instrument médical endoscopique (10),
comprenant un tuyau allongé (14) dans lequel s'étend un fil métallique (15) mobile dans le sens axial et en rotation,
comprenant un élément d'outil (17) maintenu de façon réglable, qui est relié à l'extrémité distale du fil métallique (15) ou est constitué par celle-ci,
comprenant un dispositif de positionnement axial (21) qui est placé dans ou sur l'extrémité distale du tuyau (14) et agit entre le fil métallique (15) et le tuyau (14),
dans lequel
le dispositif de positionnement axial (21) présente un guide à coulisse doté d'une rainure à coulisse (24) qui est constituée de portions rectilignes se faisant suite les unes les autres,
**caractérisé en ce que**
la rainure à coulisse (24) présente des portions axiales (25, 26, 27), orientées parallèlement au fil métallique (15), et des portions périphériques (28, 29, 30, 31) orientées perpendiculairement à celles-ci, qui sont disposées en alternance les unes avec les autres et forment une rainure à méandres.

2. Instrument selon la revendication 1, **caractérisé en ce que** le fil métallique (15) est un fil creux.

3. Instrument selon l'une des revendications précédentes, **caractérisé en ce que** l'élément d'outil (17) est une électrode (19).

4. Instrument selon l'une des revendications précédentes, **caractérisé en ce que** le dispositif de positionnement axial (21) comprend un porte-coulisse (22) dans lequel est réalisée la rainure à coulisse (24), et un coulisseau (32) qui est disposé dans la rainure à coulisse (24), avec possibilité de déplacement en longueur et en rotation.

5. Instrument selon la revendication 4, **caractérisé en ce que** le porte-coulisse (22) est relié au fil métallique (15) ou est disposé dans ou sur l'extrémité distale du tuyau (14), avec possibilité de déplacement en longueur et en rotation.

6. Instrument selon la revendication 5, **caractérisé en ce que** le coulisseau (32) est disposé de manière fixe dans ou sur l'extrémité distale du tuyau (14).

7. Instrument selon l'une des revendications précédentes, **caractérisé en ce que** le porte-coulisse (22) est relié de manière fixe à l'extrémité distale du tuyau (14).

8. Instrument selon la revendication 1, **caractérisé en ce que** la rainure à coulisse (24) est réalisée dans le tuyau (14).

9. Instrument selon la revendication 4, **caractérisé en ce que** le coulisseau (32) est relié au fil métallique et est disposé dans l'extrémité distale du tuyau (14), avec possibilité de déplacement en longueur et en rotation.

10. Instrument selon la revendication 1, **caractérisé en ce que** toutes les portions axiales (25, 26, 27) sont disposées de manière à être alignées les unes avec les autres.

11. Instrument selon la revendication 1, **caractérisé en ce que** la rainure à coulisse (24) présente au moins un élément d'arrêt (43).

12. Instrument selon la revendication 1, **caractérisé en ce que** toutes les portions périphériques (28, 29, 30, 31) sont réalisées avec une longueur uniforme.
